# EUROPÄISCHE PATENTSCHRIFT

(11) **EP 4 110 249 B1**
(45) Veröffentlichungstag und Bekanntmachung des Hinweises auf die Patenterteilung: **18.12.2024**
(21) Anmeldenummer: 21708194.2
(22) Anmeldetag: 24.02.2021
(51) Int. Cl.: A61F 9/009

(54) **SYSTEM, KONTAKTVORRICHTUNG UND VERFAHREN ZUM HERSTELLEN EINER KONTAKTVORRICHTUNG**
SYSTEM, CONTACT DEVICE, AND METHOD FOR PRODUCING A CONTACT DEVICE
SYSTÈME, DISPOSITIF DE CONTACT ET PROCÉDÉ DE FABRICATION D'UN DISPOSITIF DE CONTACT

(30) Priorität: 28.02.2020 DE 102020105335
(43) Veröffentlichungstag der Anmeldung: 04.01.2023
(73) Patentinhaber: Carl Zeiss Meditec AG, 07745 Jena (DE)
(72) Erfinder: PREUSS, Dirk, 07751 Jena (DE); WIRTH, Andreas, 84359 Simbach am Inn (DE)
(74) Vertreter: Wenzel Nemetzade Warthmüller Patentanwälte Part mbB
(86) Internationale Anmeldenummer: PCT/EP2021/054575
(87) Internationale Veröffentlichungsnummer: WO 2021/170664

(56) Entgegenhaltungen:
- WO-A1-2016/100439
- DE-A1- 102017 215 589
- JP-A- 2013 248 303
- US-B1- 6 421 173

## Beschreibung

Die Erfindung betrifft ein System, eine Kontaktvorrichtung und ein Verfahren zum Herstellen einer Kontaktvorrichtung.

### Stand der Technik

Bei einem ophthalmologischen Lasertherapiesystem wird zur Fixierung der relativen geometrischen Lage eines Patientenauges zu einem Laser-Applikator wird eine Kontaktvorrichtung in Form eines Kontaktglases oder in Form eines Flüssigkeits-Patienteninterfaces verwendet (siehe beispielsweise WO 2016/100439 A1). Bei dem Kontaktglas bzw. Flüssigkeits-Patienteninterface wird ein optisch aktives Linsenelement in der Kontaktvorrichtung an einer festen bzw. vorgegebenen Position gehalten. Hierfür wird das Linsenelement im Stand der Technik oftmals mittels Klebestoff in der Kontaktvorrichtung befestigt.

Nachteilig hieran ist, dass der Herstellungsprozess des Befestigens des Linsenelements in den Kontaktelemente zum Herstellen der Kontaktvorrichtung einen hohen Zeitaufwand und einen hohen Personalbedarf hat bzw. technisch aufwendig ist. Zudem werden hierfür spezielle Werkzeuge und besonders ausgebildetes Personal benötigt.

### Offenbarung der Erfindung

Der Erfindung liegt die Aufgabe zu Grunde, ein System bzw. eine Kontaktvorrichtung bzw. ein Verfahren zum Herstellen einer Kontaktvorrichtung aufzuzeigen, das bzw. die technisch einfach herstellbar ist bzw. das technisch einfach durchführbar ist.

Diese Aufgabe wird durch ein System gemäß Anspruch 1, eine Kontaktvorrichtung gemäß Anspruch 4 und ein Verfahren gemäß Anspruch 13 gelöst.

Insbesondere wird die Aufgabe durch ein System gelöst umfassend ein Kontaktelement zur Fixierung der relativen geometrischen Lage eines Patientenauges zu einem Laser-Applikator eines ophthalmologischen Lasertherapiesystems, und ein Linsenelement zum Einsetzen in das Kontaktelement, wobei das Kontaktelement zum Aufnehmen des Linsenelements an einer vorgegebenen Position ausgebildet ist, wobei das Linsenelement eine Augenfläche zum Berühren des Patientenauges oder zum luftdichten Begrenzen eines Hohlraums zwischen dem Linsenelement und dem Patientenauge aufweist, wobei das Linsenelement mindestens ein Vorsprungselement zum Untergreifen von einem oder mehreren Halteelementen des Kontaktelements zum klebstofflosen Befestigen des Linsenelements in dem Kontaktelement mittels einer Schnappverbindung aufweist, wobei das Linsenelement eine auf der Augenfläche zugewandten Unterseite des Vorsprungselements ausgebildete Linsenkontaktfläche aufweist, wobei das Linsenelement und das Kontaktelement derart ausgebildet sind, dass das Linsenelement derart in dem Kontaktelement mittels der Schnappverbindung befestigbar ist, dass die Linsenkontaktfläche mittels des mindestens einen Vorsprungselements und des einen Halteelements oder der mehreren Halteelemente gegen ein auf einer Befestigungsfläche des Kontaktelements angeordnetes elastisches Ausgleichselement zum Verringern der durch das eine Halteelement oder die mehreren Halteelemente auf das Linsenelement wirkenden Kräfte gedrückt wird.

Ein Vorteil hiervon ist, dass das System technisch besonders einfach herstellbar ist und das Linsenelement technisch einfach in das Kontaktelement zur Bildung einer Kontaktvorrichtung eingesetzt bzw. befestigt werden kann. Insbesondere kann das Linsenelement manuell mit einfachen allgemein erhältlichen Werkzeugen in das Kontaktelement eingesetzt und befestigt werden, um die Kontaktvorrichtung herzustellen. Auch ein automatisiertes Einsetzen bzw. Befestigen ist möglich. Zum Befestigen des Linsenelements mit dem Kontaktelement zur Herstellung der Kontaktvorrichtung wird insbesondere kein spezielles Werkzeug benötigt. Auch muss die Person, die das Linsenelement in dem Kontaktelement befestigt, nicht technisch besonders geschult bzw. ausgebildet sein. Somit kann innerhalb sehr kurzer Zeit eine große Anzahl von Kontaktvorrichtungen umfassend das Kontaktelement und das in das Kontaktelement eingesetzte bzw. in dem Kontaktelement befestigte Linsenelement hergestellt werden. Darüber hinaus ist der Platzbedarf zum Herstellen der Kontaktvorrichtung umfassend das Kontaktelement und das in das Kontaktelement eingesetzte bzw. das in dem Kontaktelement befestigte Linsenelement besonders gering. Zudem sind die Herstellungskosten gering. Darüber hinaus ist, da kein Klebstoff zum Befestigen des Linsenelements in dem Kontaktelement notwendig ist, ein unbeabsichtigtes Verkleben von anderen Elementen miteinander oder ein Anhaften von Klebstoff an nicht gewünschten Stellen sicher verhindert. Durch das Ausgleichselement wird eine Veränderung der optischen Eigenschaften des Linsenelements sicher verhindert.

Insbesondere wird die Aufgabe auch durch eine Kontaktvorrichtung zur Fixierung der relativen geometrischen Lage eines Patientenauges zu einem Laser-Applikator eines ophthalmologischen Lasertherapiesystems gelöst, wobei die Kontaktvorrichtung ein Kontaktelement und ein Linsenelement umfasst, wobei das Linsenelement klebstofflos mittels einer Schnappverbindung mit dem Kontaktelement fest verbunden ist, wobei das Linsenelement eine Augenfläche zum Berühren des Patientenauges oder zum luftdichten Begrenzen eines Hohlraums zwischen dem Linsenelement und dem Patientenauge aufweist, wobei das Linsenelement mindestens ein Vorsprungselement zum Untergreifen von einem oder mehreren Halteelementen des Kontaktelements zum klebstofflosen Befestigen des Linsenelements in dem Kontaktelement mittels einer Schnappverbindung aufweist, wobei das Linsenelement eine auf einer der Augenfläche zugewandten Unterseite des Vorsprungselements ausgebildete Linsenkontaktfläche aufweist, wobei Linsenelement derart in dem Kontaktelement mittels der Schnappverbindung befestigt ist, dass die Linsenkontaktfläche mittels des mindestens einen Vorsprungselements und des einen Halteelements oder der mehreren Halteelemente gegen ein auf einer Befestigungsfläche des Kontaktelements angeordnetes elastisches Ausgleichselement zum Verringern der durch das eine Halteelement oder die mehreren Halteelemente auf das Linsenelement wirkenden Kräfte gedrückt wird.

Vorteilhaft hieran ist, dass die Kontaktvorrichtung technisch besonders einfach herstellbar ist. Insbesondere kann das Linsenelement technisch besonders einfach in dem Kontaktelement befestigt sein. Bei der Herstellung der Kontaktvorrichtung wird kein spezielles bzw. angepasstes Werkzeug benötigt. Die Herstellung der Kontaktvorrichtung kann automatisiert bzw. maschinell durchgeführt werden. Für die Herstellung der Kontaktvorrichtung muss die Person, die das Linsenelement in dem Kontaktelement befestigt, nicht technisch besonders geschult bzw. ausgebildet sein. Somit kann innerhalb sehr kurzer Zeit eine große Anzahl von Kontaktvorrichtungen umfassend das Kontaktelement und das in das Kontaktelement eingesetzte bzw. in dem Kontaktelement befestigte Linsenelement hergestellt werden. Darüber hinaus ist der Platzbedarf zum Herstellen der Kontaktvorrichtung umfassend das Kontaktelement und das in das Kontaktelement eingesetzte bzw. das in dem Kontaktelement befestigte Linsenelement besonders gering. Zudem sind die Herstellungskosten gering. Darüber hinaus ist, da kein Klebstoff zum Befestigen des Linsenelements in dem Kontaktelement notwendig ist bzw. verwendet wird, ein unbeabsichtigtes Verkleben von anderen Elementen miteinander oder ein Anhaften von Klebstoff an nicht gewünschten Stellen sicher verhindert. Durch das Ausgleichselement wird eine Veränderung der optischen Eigenschaften des Linsenelements sicher verhindert.

Insbesondere wird die Aufgabe auch durch ein Verfahren zum Herstellen einer Kontaktvorrichtung zur Fixierung der relativen geometrischen Lage eines Patientenauges zu einem Laser-Applikator eines ophthalmologischen Lasertherapiesystems gelöst, wobei die Kontaktvorrichtung ein Kontaktelement und ein Linsenelement aufweist, wobei das Verfahren folgende Schritte umfasst:
- Bereitstellen eines Kontaktelements mit einem oder mehreren Halteelementen;
- Bereitstellen eines Linsenelements, wobei das Linsenelement eine Augenfläche zum Berühren des Patientenauges oder zum luftdichten Begrenzen eines Hohlraums zwischen dem Linsenelement und dem Patientenauge aufweist, wobei das Linsenelement mindestens ein Vorsprungselement zum Untergreifen des einen oder der mehreren Halteelemente des Kontaktelements zum klebstofflosen Befestigen des Linsenelements in dem Kontaktelement mittels einer Schnappverbindung aufweist, wobei das Linsenelement eine auf der Augenfläche zugewandten Unterseite des mindestens einen Vorsprungselements ausgebildete Linsenkontaktfläche aufweist; und
- klebstoffloses Befestigen des Linsenelements in dem Kontaktelement mittels einer Schnappverbindung, indem das mindestens eine Vorsprungselement unter dem oder den Halteelementen derart eingeschnappt wird, dass die Linsenkontaktfläche mittels des mindestens einen Vorsprungselements und des einen Halteelements oder der mehreren Halteelementen gegen ein auf einer Befestigungsfläche des Kontaktelements angeordnetes elastisches Ausgleichselement zum Verringern der durch das eine Halteelement oder die mehreren Halteelemente auf das Linsenelement wirkenden Kräfte gedrückt wird.

Ein Vorteil dieses Verfahrens ist, dass das Verfahren technisch einfach und schnell durchführbar ist. Zudem braucht das Verfahren wenig Raum bzw. Platz. Darüber hinaus werden keine speziell angepassten Werkzeuge benötigt. Das Personal, das das Verfahren ausführt, muss nicht speziell geschult sein. Folglich ist das Verfahren kostengünstig durchführbar. Durch das Ausgleichselement wird eine Veränderung der optischen Eigenschaften des Linsenelements sicher verhindert. Darüber hinaus wird, da kein Klebstoff zum festen Verbinden des Linsenelements mit dem Kontaktelement verwendet wird, ein unbeabsichtigtes Verkleben von anderen Elementen miteinander oder ein Anhaften von Klebstoff an nicht gewünschten Stellen sicher verhindert.

Gemäß einer Ausführungsform des Systems sind das Linsenelement und das Kontaktelement derart ausgebildet, dass das Linsenelement derart in dem Kontaktelement befestigbar ist, dass das Ausgleichselement um die optische Achse des Linsenelements vollständig umlaufend angeordnet ist. Vorteilhaft hieran ist, dass das die auftretenden Kräfte besonders gleichmäßig über das Linsenelement und die Befestigungsfläche verteilt werden. Dies verhindert negative Auswirkungen auf die optischen Eigenschaften des Linsenelements noch sicherer.

Gemäß einer Ausführungsform des Systems ist das Ausgleichselement zum luftdichten Abdichten des Bereichs zwischen der Linsenkontaktfläche und der Befestigungsfläche ausgebildet. Ein Vorteil hiervon ist, dass technisch einfach eine Kontaktvorrichtung, die als Flüssigkeits-Patienteninterface an dieser Stelle abdichtet, oder ein Kontaktglas, das über die gesamte Kontaktfläche angesaugt wird, ausgebildet bzw. herstellbar ist. Das Ausgleichselement kann somit gleichzeitig die Funktion eines Dichtungselements übernehmen. Es ist möglich, dass das Linsenelement derart in dem Kontaktelement befestigbar ist, dass das Ausgleichselement um die optische Achse des Linsenelements nicht vollständig umlaufend angeordnet ist und dennoch beim Aufsetzen auf das Patientenauge bzw. beim Ansaugen an dem Patientenauge der Bereich zwischen der Linsenkontaktfläche und der Befestigungsfläche im Wesentlichen dichtend oder im Wesentlichen luftdicht abgedichtet wird. Beispielsweise kann durch das Ansaugen an dem Patientenauge ein Teil der Linsenkontaktfläche mit einem Teil der Befestigungsfläche, in dem kein Ausgleichselement vorhanden ist, im Wesentlichen dichtend bzw. im Wesentlichen luftdicht miteinander verbunden werden. Das Ansaugsystem kann die Aussparungen zwischen den zueinander beabstandeten Ausgleichselementen kompensieren.

Gemäß einer Ausführungsform der Kontaktvorrichtung ist das Linsenelement derart in dem Kontaktelement befestigt, dass das Ausgleichselement um die optische Achse des Linsenelements vollständig umlaufend angeordnet ist. Ein Vorteil hiervon ist, dass bei der Kontaktvorrichtung die auftretenden Kräfte besonders gleichmäßig über das Linsenelement und die Befestigungsfläche verteilt und Spannungen vermieden werden. Dies verhindert negative Auswirkungen auf die optischen Eigenschaften des Linsenelements noch sicherer.

Gemäß einer Ausführungsform des Systems oder der Kontaktvorrichtung verläuft die Linsenkontaktfläche senkrecht zur optischen Achse des Linsenelements. Ein Vorteil hiervon ist, dass die auf das Linsenelement wirkenden Kräfte besonders effizient und großflächig verteilt werden können.

Gemäß einer Ausführungsform des Systems oder der Kontaktvorrichtung verläuft die Befestigungsfläche des Kontaktelements senkrecht zur optischen Achse des Linsenelements, wenn das Linsenelement in dem Kontaktelement befestigt ist. Hierdurch können auftretende Kräfte durch die Schnappverbindung großflächig und effizient in das Kontaktelement übertragen werden. Somit wird zuverlässig verhindert, dass sich die optischen Eigenschaften des Linsenelements verändern. Zudem kann, wenn die Linsenkontaktfläche parallel zu der Befestigungsfläche angeordnet ist, das Ausgleichselement großflächig mit der Linsenkontaktfläche und der Befestigungsfläche in Kontakt stehen.

Gemäß einer Ausführungsform des Systems oder der Kontaktvorrichtung umfasst oder ist das mindestens eine Vorsprungselement des Linsenelements eine im Wesentlichen vollständig um die optische Achse des Linsenelements umlaufende Wulst oder das mindestens eine Vorsprungselement weist mehrere um die optische Achse der Linse herum zueinander beabstandet angeordnete Vorsprungselemente auf. Vorteilhaft an einer umlaufenden Wulst ist, dass das Linsenelement bzw. das System bzw. die Kontaktvorrichtung technisch noch einfacher herstellbar ist. Zudem können bei einer umlaufenden Wulst das Halteelement oder die Halteelemente das Linsenelement besonders großflächig sichern und an einer vorgegebenen Position halten. Darüber hinaus kann bei einer umlaufenden Wulst das Linsenelement um die optische Achse des Linsenelements relativ zu dem Kontaktelement gedreht werden. Vorteilhaft an mehreren zueinander beabstandeten Vorsprungselementen ist, dass das Linsenelement technisch besonders einfach in dem Kontaktelement befestigt bzw. eingesetzt werden kann.

Gemäß einer Ausführungsform des Systems oder der Kontaktvorrichtung ist das Ausgleichselement in einem Querschnitt senkrecht zur optischen Achse des Linsenelements im Wesentlichen kreisringförmig ausgebildet, wenn das Linsenelement in dem Kontaktelement befestigt ist. Ein Vorteil hiervon ist, dass das Ausgleichselement technisch besonders einfach anordenbar ist. Dies verringert die Herstellungskosten und verringert die für das Herstellen benötigte Zeit. Zudem können die Kräfte besonders gleichmäßig von dem Linsenelement in das Ausgleichselement übertragen werden. Dies verhindert mögliche negative Auswirkungen auf optische Eigenschaften des Linsenelements noch sicherer.

Gemäß einer Ausführungsform des Systems oder der Kontaktvorrichtung umfasst das Kontaktelement mehrere Halteelemente, die, insbesondere äquidistant zueinander, um die optische Achse der Linse herum beabstandet zueinander angeordnet sind, wenn das Linsenelement in dem Kontaktelement befestigt ist. Vorteilhaft hieran ist, dass das Linsenelement technisch einfach in das Kontaktelement eingesetzt werden kann. Zudem treten auch beim Einsetzen des Linsenelements in das Kontaktelement bzw. beim Herstellen der Schnappverbindung keine großen Kräfte auf das Linsenelement auf, so dass eine Veränderung der optischen Eigenschaften des Linsenelements auch beim Einsetzen in das Kontaktelement sicher verhindert ist.

Gemäß einer Ausführungsform des Systems oder der Kontaktvorrichtung ist/sind das eine oder die mehreren Halteelemente des Kontaktelements derart flexibel ausgebildet, dass das eine oder die mehreren Halteelemente mechanisch beweglich zum Einrasten des mindestens einen Vorsprungs des Linsenelements unter dem Halteelement oder den Halteelementen zum Bilden der Schnappverbindung ausgebildet ist/sind. Hierdurch kann das Linsenelement technisch einfach, insbesondere manuell, in das Kontaktelement eingesetzt werden. Zudem wirken auch während des Einsetzens des Linsenelements in das Kontaktelement nur geringe Kräfte auf das Linsenelement. Hierdurch wird eine (dauerhafte) Veränderung der optischen Eigenschaften des Linsenelements sicher verhindert.

Gemäß einer Ausführungsform des Systems oder der Kontaktvorrichtung weist das Ausgleichselement eine der jeweiligen Bauart der Kontaktvorrichtung zugeordnete Markierung und/oder Farbe auf. Vorteilhaft hieran ist, dass der Mensch optisch schnell und sicher unterschiedliche Bauarten der Kontaktvorrichtung unterscheiden kann. Somit wird eine Fehlbenutzung bzw. Fehlauswahl der Kontaktvorrichtung sicher vermieden. Unterschiedliche Bauarten der Kontaktvorrichtung können beispielsweise jeweils unterschiedliche Linsenelemente und jeweils baugleiche Kontaktelemente umfassen.

Gemäß einer Ausführungsform des Verfahrens verläuft die Linsenkontaktfläche senkrecht zur optischen Achse des Linsenelements. Vorteilhaft hieran ist, dass die auf das Linsenelement wirkenden Kräfte besonders effizient und großflächig verteilt werden.

Gemäß einer Ausführungsform des Verfahrens wird das Kontaktelement umfassend das flexible Ausgleichselement mittels Mehrkomponenten-Spritzgussverfahren, insbesondere Zweikomponenten-Spritzgussverfahren, hergestellt. Vorteilhaft hieran ist, dass die Kontaktvorrichtung technisch einfach, schnell und kostengünstig herstellbar ist. Zudem wird kein zusätzlicher Arbeitsschritt zum Anordnen des Ausgleichselements benötigt.

Gemäß einer Ausführungsform des Verfahrens wird/werden das eine oder die mehreren Halteelemente des Kontaktelements mechanisch, insbesondere von der optischen Achse des Linsenelements weg, reversibel bewegt zum Einführen eines Teils des mindestens einen Vorsprungs des Linsenelements zwischen das Halteelement oder die Halteelemente des Kontaktelements und die Befestigungsfläche des Kontaktelements zur Bildung der Schnappverbindung. Ein Vorteil hiervon ist, dass das Linsenelement technisch einfach, insbesondere manuell, in das Kontaktelement einsetzbar ist. Zudem wirken auch während des Einsetzens nur geringe Kräfte auf das Linsenelement. Hierdurch wird eine (dauerhafte) Veränderung der optischen Eigenschaften des Linsenelements sicher verhindert.

Gemäß einer Ausführungsform des Verfahrens umfasst das mindestens eine Vorsprungselement des Linsenelements eine im Wesentlichen vollständig um die optische Achse des Linsenelements umlaufende Wulst. Ein Vorteil hiervon ist, dass die Kontaktvorrichtung technisch noch einfacher herstellbar ist. Auch wird das Linsenelement durch das Halteelement oder die Halteelement besonders großflächig gesichert und an einer vorgegebenen Position gehalten. Zudem verändert eine Drehung des Linsenelements um die optische Achse des Linsenelements relativ zu dem Kontaktelement die Befestigung des Linsenelements in dem Kontaktelement nicht.

Es ist vorstellbar, dass das Kontaktelement eine oder mehrere Aussparungen aufweist, in die das Ausgleichselement beim Einsetzen des Linsenelements in das Kontaktelement zumindest teilweise gedrückt wird und in die das Ausgleichselement sozusagen teilweise ausweichen kann. Hierdurch werden die auf das Linsenelement wirkenden Kräfte noch weiter verringert.

Bevorzugte Ausführungsformen ergeben sich aus den Unteransprüchen. Nachfolgend wird die Erfindung anhand von Zeichnungen von Ausführungsbeispielen näher erläutert. Hierbei zeigen
- Fig. 1: eine schematische Seitenansicht einer Ausführungsform der erfindungsgemäßen Kontaktvorrichtung;
- Fig. 2: eine Detailansicht des Bereichs II aus Fig. 1; und
- Fig. 3: eine Aufsicht auf das Kontaktelement der Kontaktvorrichtung aus Fig. 1 bzw. Fig. 2.

Bei der nachfolgenden Beschreibung werden für gleiche und gleich wirkende Teile dieselben Bezugsziffern verwendet.

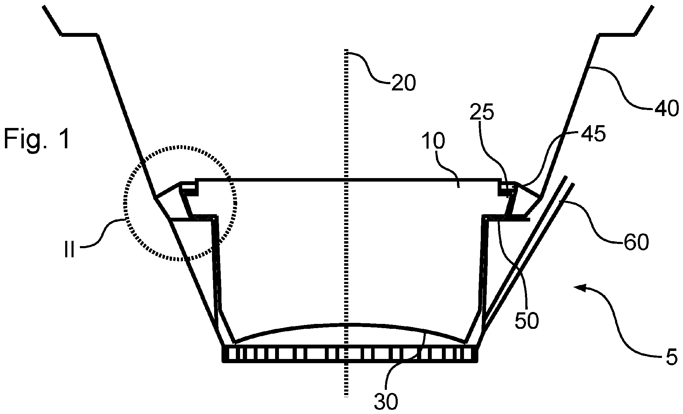

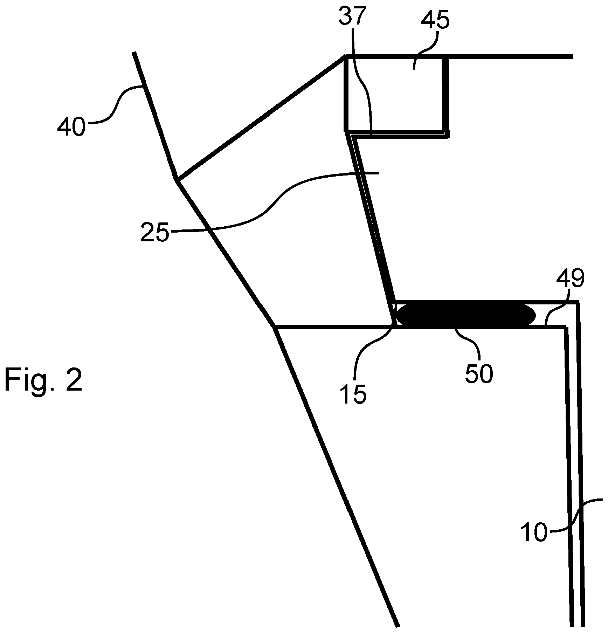

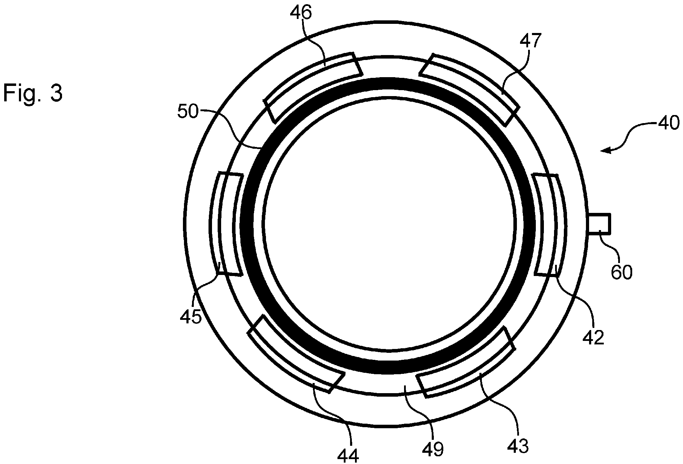

Fig. 1 zeigt eine schematische Seitenansicht einer Ausführungsform der erfindungsgemäßen Kontaktvorrichtung 5. Fig. 2 zeigt eine Detailansicht des Bereichs II aus Fig. 1. Fig. 3 zeigt eine Aufsicht auf das Kontaktelement 40 der Kontaktvorrichtung 5 aus Fig. 1 bzw. Fig. 2 von oben, d.h. in die Richtung des Lichtstrahls während der Behandlung des Patientenauges.

Die Kontaktvorrichtung 5 ist zur Fixierung der relativen geometrischen Lage eines Patientenauges zu einem Laser-Applikator eines ophthalmologischen Lasertherapiesystems ausgebildet. Die Kontaktvorrichtung 5 fixiert somit die Position des Auges in Bezug auf die Laserstrahlung während der Behandlung mit Laserstrahlung. Die Kontaktvorrichtung 5 ist bzw. wird starr/fest am Lasertherapiesystem befestigt.

Die Kontaktvorrichtung 5 umfasst ein Linsenelement 10, das mittels Schnappverbindung klebstofflos in dem Kontaktelement 40 der Kontaktvorrichtung 5 an bzw. in einer vorgegebenen Position befestigt ist.

Die Kontaktvorrichtung 5 kann eine Kontaktglashaltevorrichtung (umgangssprachlich auch einfach "Kontaktglas" genannt) sein, bei der das Linsenelement 10 das Auge bzw. die Cornea berührt. Es ist möglich, dass die Kontaktvorrichtung 5 ein Flüssigkeits-Patienteninterface ist, bei dem ein Hohlraum zwischen dem Linsenelement 10 und dem Patientenauge mit einer physiologischen Salzlösung (engl. buffered salt solution, Abkürzung: BSS) befüllt wird. Durch die Salzlösung kann der Brechungsindex zwischen dem Linsenelement 10 und dem Patientenauge angepasst werden. Insbesondere bei der Fokussierung der Laserstrahlung auf tieferliegende Strukturen im Patientenauge kann ein Flüssigkeits-Patienteninterface verwendet werden.

Das Linsenelement 10 ist fest bzw. an einer vorgegebenen Position in dem Kontaktelement 40 der Kontaktvorrichtung 5 befestigt bzw. angeordnet. Dies bedeutet, dass die relative Position zwischen dem Linsenelement 10 und dem Kontaktelement 40 im Wesentlichen unveränderbar ist.

Das Linsenelement 10 ist mittels einer Schnappverbindung bzw. Klickverbindung fest in das Kontaktelement 40 eingesetzt, d.h. mit dem Kontaktelement 40 fest verbunden bzw. in dem Kontaktelement 40 befestigt. Die Schnappverbindung bildet einen Formschluss aus.

Die optische Achse 20 des Linsenelements 10 verläuft zentrisch durch das Linsenelement 10, d.h. in Fig. 1 bzw. Fig. 2 von oben nach unten. In Fig. 3 verläuft die optische Achse 20 des Linsenelements 10 senkrecht zur Zeichenebene, wenn das Linsenelement 10 an der vorgegebenen Position in dem Kontaktelement 40 befestigt ist bzw. wird.

Zum Herstellen der Schnappverbindung zwischen dem Linsenelement 10 und dem Kontaktelement 40 weist das Linsenelement 10 ein Vorsprungselement 25, z.B. in Form einer umlaufenden Wulst, oder mehrere Vorsprungselemente 25 auf. Die mehreren Vorsprungselemente 25 können um die optische Achse 20 herum zueinander in Umfangsrichtung beabstandet ausgebildet sein. Beispielsweise können die mehreren Vorsprungselemente 25 in Umfangsrichtung um die optische Achse 20 herum äquidistant zueinander beabstandet sein. So können beispielswiese sechs Vorsprungselemente 25, die sich jeweils über 30° des Vollkreises bzw. Umfangs erstrecken, jeweils einen Abstand von 30° in Umfangsrichtung zueinander aufweisen. Andere Ausbildungen von Vorsprungselementen 25 sind vorstellbar.

Das Kontaktelement 40 weist eine kegelstumpfförmige Außenform auf. Das Kontaktelement 40 weist auf seiner Innenseite ein Halteelement 42 oder mehrere Halteelemente 42-47 auf. Das Halteelement 42 oder die Halteelemente 42-47 sind in Form von Vorsprüngen nach innen bzw. zur optischen Achse 20 des Linsenelements 10 hin ausgebildet.

Das Halteelement 42-47 kann vollständig um die optische Achse 20 des Linsenelements 10 umlaufend ausgebildet sein, d.h. sich über den Vollkreis um die optische Achse 20 des Linsenelements 10 erstrecken. Alternativ ist es möglich, dass mehrere Halteelemente 42-47 in Umfangsrichtung zueinander beabstandet, insbesondere äquidistant, angeordnet sind. Wie die Vorsprungselemente 25 können die Halteelemente 42-47 sich jeweils über einen Kreisbogensegment erstrecken, z.B. über 35° des Umfangs. Alternativ können auch drei Halteelemente 42-47 vorhanden sein, die jeweils um 120° zueinander versetzt in Umfangsrichtung angeordnet sind.

Das Kontaktelement 40 weist eine Befestigungsfläche 49 auf. Das Linsenelement 10 weist auf der dem Auge zugewandten Seite eine Linsenkontaktfläche 15 auf. Die Linsenkontaktfläche 15 kann senkrecht zur optischen Achse 20 des Linsenelements 10 verlaufen. Die Befestigungsfläche 49 kann senkrecht zur optischen Achse 20 des Linsenelements 10 ausgebildet sein. Wenn das Linsenelement 10 in das Kontaktelement 40 eingesetzt ist und mittels Schnappverbindung befestigt ist, verlaufen die Linsenkontaktfläche 15 und die Befestigungsfläche 49 parallel zueinander.

Das Linsenelement 10 ist klebstofflos bzw. ohne Klebstoff in dem Halteelement 42-47 befestigt. Insbesondere befindet sich zwischen der Linsenkontaktfläche 15 und der Befestigungsfläche 49 kein Klebstoff.

Das Linsenelement 10 wird in dem Kontaktelement 40 befestigt, indem das Vorsprungselement 25 oder die Vorsprungselemente des Linsenelements 10 unter dem Halteelement 42-47 oder den Halteelementen 42-47 des Kontaktelements 40 eingeführt werden und einschnappen. Hierfür können die Vorsprungselemente 25 oder die Halteelemente 42-47 reversibel mechanisch bewegbar sein. Insbesondere können die Vorsprungselemente 25 und/oder die Halteelemente 42-47 von der optischen Achse 20 des Linsenelements 10 weg bzw. hin reversibel beweglich sein. Durch Einführung des Vorsprungselements 25 bzw. der Vorsprungselemente 25 unter das Halteelement 42-47 oder die Halteelemente 42-47 wird die Schnappverbindung bzw. Klickverbindung zwischen dem Linsenelement 10 und dem Kontaktelement 40 gebildet. Somit wird ein Formschluss zwischen Halteelement 42-47 und Vorsprungselement 25 gebildet, der ein Bewegen des Linsenelements 10 gegenüber dem Kontaktelement 40 im Wesentlichen verhindert.

Das Vorsprungselement 25 wird zwischen dem Halteelement 42-47 bzw. den Halteelementen 42-47 und der Befestigungsfläche 49 gehalten. Das Vorsprungselement 25 und das Halteelement 42-47 drücken die Linsenkontaktfläche 15 in Richtung der Befestigungsfläche 49.

Auf der Befestigungsfläche 49 ist ein elastisches Ausgleichselement 50 angeordnet. Das Ausgleichselement 50 ist elastisch ausgebildet, d.h. das Ausgleichselement 50 strebt danach seine Ursprungsform wieder anzunehmen. Das Ausgleichselement 50 kann einen Kunststoff umfassen oder aus Kunststoff bestehen. Beispielsweise besteht das Ausgleichselement 50 aus einem Gummikunststoff.

Es ist möglich, aber nicht zwingend, dass in der Befestigungsfläche 49 eine Aussparung bzw. Vertiefung zum Aufnehmen eines Teils des Ausgleichselements 50 ausgebildet ist. Auf diese Weise kann das Ausgleichselement 50 besonders präzise angeordnet werden.

Das Ausgleichselement 50 kann in einer Querschnittsebene, die senkrecht zur optischen Achse 20 des Linsenelements 10 verläuft, ringförmig ausgebildet sein. Das Ausgleichselement 50 kann torusförmig ausgebildet sein, ähnlich eines O-Rings. Andere Formen sind möglich. Z.B. kann es eine Donutform aufweisen.

Das Ausgleichselement 50 kann vollständig um die optische Achse 20 des Linsenelements 10 umlaufend ausgebildet sein.

Das Ausgleichselement 50 kann luftdicht ausgebildet sein. Hierdurch kann der Bereich zwischen der Linsenkontaktfläche 15 und der Befestigungsfläche 49 luftdicht abgeschlossen werden. Dies ist insbesondere von Bedeutung bei einem Flüssigkeit-Patienteninterface, da hier der Hohlraum, der von der Augenfläche 30 des Linsenelements 10 begrenzte Hohlraum zwischen Linsenelement 10 und Patientenauge dichtend abgeschlossen sein sollte. Ebenso von Bedeutung ist dies bei einem Kontaktelement, das als Kontaktglas ausgebildet ist und über die gesamte Kontaktfläche mit dem Auge angesaugt wird. Möglich ist jedoch auch dass, insbesondere bei einer Ausbildung als Flüssigkeits-Patienteninterface, ein zusätzlicher Ansaugring, beispielsweise am Außenumfang bzw. in der Nähe des Außenumfangs des Kontaktelements 40, vorhanden ist, wobei der Ansaugring zum Ansaugen des Patientenauges und somit zur relativen Fixierung des Kontaktelements 40 gegenüber dem Patientenauge ausgebildet ist. Bei einem zusätzlichen Ansaugring ist beispielsweise keine luftdichte Abschließung des Hohlraums zwischen Linsenelement 10 und Patientenauge notwendig.

Denkbar ist auch, dass das Ausgleichselement 50 mehrere zueinander beabstandete Segmente aufweist, die über den Umfang der Befestigungsfläche 49 in Umfangsrichtung, insbesondere äquidistant, zueinander beabstandet sind. Beispielsweise befindet sich an 6 Punkten der Befestigungsfläche 49 ein Segment des Ausgleichselements 50. Die verschiedenen Segmente des Ausgleichselements 50 weisen typischerweise dieselbe Elastizität auf. Bei mehreren zueinander beabstandeten Segmenten des Ausgleichselements 50 bzw. bei mehreren zueinander beabstandeten Ausgleichselementen ist es denkbar, dass durch den Druck der Linsenkontaktfläche 15 gegen die Befestigungsfläche 49 ein dichtender Verschluss bzw. ein luftdichter Verschluss des Bereichs zwischen der Linsenkontaktfläche 15 und der Befestigungsfläche 49 gebildet wird.

Das Kontaktelement 40 ist zumindest teilweise komplementär zu dem Linsenelement 10 ausgebildet, insbesondere derart, dass eine laterale Bewegung, d.h. senkrecht zur optischen Achse 20 des Linsenelements 10 verlaufende Bewegung, im Wesentlichen nicht möglich ist, wenn das Linsenelement 10 mittels der Schnappverbindung mit dem Kontaktelement 40 fest verbunden bzw. in dem Kontaktelement 40 befestigt ist. In lateraler Richtung bzw. seitlicher Richtung liegt somit ein Passsitz vor. In lateraler Richtung bzw. seitlicher Richtung wirken somit nicht ständig Kräfte auf das Linsenelement 10.

Denkbar ist jedoch, dass auch in lateraler Richtung zwischen dem Linsenelement 10 und dem Kontaktelement 40 ein weiteres Ausgleichselement 50 angeordnet ist. Das weitere Ausgleichselement 50 kann über den Vollkreis um die optische Achse 20 des Linsenelements 10 umlaufend sein oder nur aus einzelnen Kreissegmenten oder Bogensegmenten bestehen.

Das Ausgleichselement 50 wird sozusagen zwischen der Linsenkontaktfläche 15 und der Befestigungsfläche 49 eingeklemmt. Denkbar ist, dass das Ausgleichselement 50 den größten Teil, insbesondere mehr als 90% der Linsenkontaktfläche 15 und/oder der Befestigungsfläche 49 bedeckt.

Das Ausgleichselement 50 verhindert, dass zu große Kräfte auf das Linsenelement 10 wirken bzw. Spannungen in dem Linsenelement 10 entstehen, die die optischen Eigenschaften des Linsenelements 10 verändern könnten. Das Drücken der Linsenkontaktfläche 15 direkt gegen die Befestigungsfläche 49 wird durch das Ausgleichselement 50 verhindert.

Das Ausgleichselement 50 umfasst keinen Klebstoff und übt keine klebende Funktion aus.

Das Ausgleichselement 50 verformt sich durch das Drücken des Linsenelements 10 bzw. der Linsenkontaktfläche 15 in Richtung der Befestigungsfläche 49. Das Ausgleichselement 50 drückt entsprechend aufgrund seiner Elastizität gegen die Linsenkontaktfläche 15. Hierdurch wird eine Bewegung des Linsenelements 10 parallel zu der optischen Achse 20 verhindert. Das Ausgleichselement 50 stellt somit eine Art Puffer zwischen Linsenkontaktfläche 15 und Befestigungsfläche 49 dar, der Kräfte durch Verformung aufnehmen kann, so dass zwischen der im Wesentlichen nicht verformbaren Linsenkontaktfläche 15 und der im Wesentlichen nicht verformbaren Befestigungsfläche 49 üblicherweise kein direkter bzw. unmittelbarer Kontakt hergestellt wird.

Das Kontaktelement 40 mit dem Ausgleichselement 50 kann mittels Spritzgussverfahren hergestellt sein bzw. werden. Insbesondere kann das Kontaktelement 40 mittels eines 2-Komponenenten-Spritzgussverfahrens bzw. 2K-Spritzgussverfahrens hergestellt sein bzw. werden. Das Kontaktelement 40 ohne das Ausgleichselement 50 kann aus einer ersten Komponente bestehen bzw. hergestellt werden und das Ausgleichselement 50 kann aus einer zweiten Komponente bestehen bzw. hergestellt werden, wobei sich die zweite Komponente von der ersten Komponente unterscheidet. Die zweite Komponente kann einen elastischen bzw. flexiblen Kunststoff bzw. Kunststoffgemisch umfassen oder sein.

Eine der Augenfläche 30 gegenüberliegende Therapiesystemfläche 37 ist parallel zu der Augenfläche 30 ausgebildet. Die Therapiesystemfläche 37 berührt bzw. kontaktiert das Halteelement 42-47 des Kontaktelements 40. Zwischen der Therapiesystemfläche 37 und dem Halteelement 42-47 liegt ein Formschluss vor. Die Therapiesystemfläche 37 kann sich in Fig. 1 bzw. Fig. 2 nicht nach oben bewegen, da die Therapiesystemfläche 37 gegen das Halteelement 42-47 des Kontaktelements 40 stößt.

Die Therapiesystemfläche 37 kann der Augenfläche 30 abgewandt sein. Dies bedeutet, dass die Augenfläche 30 in Fig. 1 bzw. Fig. 2 nach unten zeigt, während die Therapiesystemfläche 37 nach oben zeigt bzw. auf der Oberseite des Vorsprungs des Linsenelements 10 ausgebildet ist. Die Linsenkontaktfläche 15 ist auf der Unterseite des Vorsprungs des Linsenelements 10 ausgebildet.

Das Linsenelement 10 wird in das Kontaktelement 40 zum Bilden der Kontaktvorrichtung 5 eingesetzt, indem eines oder mehrere Halteelemente 42-47 reversibel mechanisch bewegt werden. Beispielsweise kann ein Haltelement oder können mehrere Halteelemente 42-47 lateral bzw. seitlich und/oder parallel zu der optischen Achse 20 des Linsenelements 10 teilweise gebogen werden, um das Vorsprungselement 25 bzw. die Vorsprungselemente unter das Halteelement 42-47 bzw. die Halteelemente 42-47 zu bewegen. Anschließend bewegt sich bzw. schnappt das Halteelement 42-47 oder die Halteelemente 42-47 wieder zurück an ihre ursprüngliche Position(en). Nun ist eine formschlüssige Schnappverbindung zwischen Linsenelement 10 und Kontaktelement 40 gebildet. Das Vorsprungselement 25 bzw. die Vorsprungselemente des Linsenelements 10 befindet/befinden sich nun zwischen dem Halteelement 42-47 bzw. den Halteelementen 42-47 und der Befestigungsfläche 49. Zwischen dem Vorsprungselement 25 und der Befestigungsfläche 49 ist das Ausgleichselement 50 angeordnet.

Denkbar ist auch, dass ein Vorsprungselement 25 oder mehrere Vorsprungselemente des Linsenelements 10 mechanisch reversibel bewegt werden, um das Linsenelement 10 in das Kontaktelement 40 einzusetzen und in dem Kontaktelement 40 mittels Schnappverbindung bzw. Klickverbindung zu befestigen.

Die Schnappverbindung kann typischerweise nicht zerstörungsfrei aus dem Kontaktelement 40 gelöst werden.

In Fig. 3 ist das Linsenelement 10 noch nicht in das Kontaktelement 40 eingesetzt.

Es ist möglich, dass die gesamte Kontaktvorrichtung 5 keinen Klebstoff bzw. keine Klebstoffverbindung aufweist.

Die Kontaktvorrichtung 5 kann einen Ansaugkanal 60 zum Ansaugen des Auges aufweisen.

### Bezugszeichenliste

- 5: Kontaktvorrichtung
- 10: Linsenelement
- 15: Linsenkontaktfläche
- 20: optische Achse der Linse
- 25: Vorsprungselement
- 30: Augenfläche
- 37: Therapiesystemfläche
- 40: Kontaktelement
- 42-47: Halteelement
- 49: Befestigungsfläche
- 50: Ausgleichselement
- 60: Ansaugkanal

## Patentansprüche

1. System umfassend
ein Kontaktelement (40) zur Fixierung der relativen geometrischen Lage eines Patientenauges zu einem Laser-Applikator eines ophthalmologischen Lasertherapiesystems,
und
ein Linsenelement (10) zum Einsetzen in das Kontaktelement (40),
wobei das Kontaktelement (40) zum Aufnehmen des Linsenelements (10) an einer vorgegebenen Position ausgebildet ist,
wobei das Linsenelement (10) eine Augenfläche (30) zum Berühren des Patientenauges oder zum luftdichten Begrenzen eines Hohlraums zwischen dem Linsenelement (10) und dem Patientenauge aufweist,
wobei das Linsenelement (10) mindestens ein Vorsprungselement (25) zum Untergreifen von einem oder mehreren Halteelementen (42-47) des Kontaktelements (40) zum klebstofflosen Befestigen des Linsenelements (10) in dem Kontaktelement (40) mittels einer Schnappverbindung aufweist, wobei das Linsenelement (10) eine auf einer der Augenfläche (30) zugewandten Unterseite des Vorsprungselements (25) ausgebildete Linsenkontaktfläche (15) aufweist,
wobei das Linsenelement (10) und das Kontaktelement (40) derart ausgebildet sind, dass das Linsenelement (10) derart in dem Kontaktelement (40) mittels der Schnappverbindung befestigbar ist, dass die Linsenkontaktfläche (15) mittels des mindestens einen Vorsprungselements (25) und des einen Halteelements (42-47) oder der mehreren Halteelemente (42-47) gegen ein auf einer Befestigungsfläche (49) des Kontaktelements (40) angeordnetes elastisches Ausgleichselement (50) zum Verringern der durch das eine Halteelement (42-47) oder die mehreren Halteelemente (42-47) auf das Linsenelement (10) wirkenden Kräfte gedrückt wird.

2. System nach Anspruch 1, wobei
das Linsenelement (10) und das Kontaktelement (40) derart ausgebildet sind, dass das Linsenelement (10) derart in dem Kontaktelement (40) befestigbar ist, dass das Ausgleichselement (50) um die optische Achse (20) des Linsenelements (10) vollständig umlaufend angeordnet ist.

3. System nach Anspruch 1 oder 2, wobei
das Ausgleichselement (50) zum luftdichten Abdichten des Bereichs zwischen der Linsenkontaktfläche (15) und der Befestigungsfläche (49) ausgebildet ist.

4. Kontaktvorrichtung (5),
wobei die Kontaktvorrichtung (5) ein System nach einem der Ansprüche 1-3 umfasst,
wobei die Kontaktvorrichtung (5) zur Fixierung der relativen geometrischen Lage des Patientenauges zu dem Laser-Applikator des ophthalmologischen Lasertherapiesystems ausgebildet ist,
wobei das Linsenelement (10) klebstofflos mittels der Schnappverbindung mit dem Kontaktelement (40) fest verbunden ist,
wobei Linsenelement (10) derart in dem Kontaktelement (40) mittels der Schnappverbindung befestigt ist, dass die Linsenkontaktfläche (15) mittels des mindestens einen Vorsprungselements (25) und des einen Halteelements (42-47) oder der mehreren Halteelemente (42-47) gegen das auf der Befestigungsfläche (49) des Kontaktelements (40) angeordnete elastische Ausgleichselement (50) zum Verringern der durch das eine Halteelement (42-47) oder die mehreren Halteelemente (42-47) auf das Linsenelement (10) wirkenden Kräfte gedrückt wird.

5. Kontaktvorrichtung (5) nach Anspruch 4, wobei
das Linsenelement (10) derart in dem Kontaktelement (40) befestigt ist, dass das Ausgleichselement (50) um die optische Achse (20) des Linsenelements (10) vollständig umlaufend angeordnet ist.

6. System nach einem der Ansprüche 1-3 oder Kontaktvorrichtung (5) nach Anspruch 4 oder Anspruch 5, wobei
die Linsenkontaktfläche (15) senkrecht zur optischen Achse (20) des Linsenelements (10) verläuft.

7. System nach einem der Ansprüche 1-3 oder Kontaktvorrichtung (5) nach einem der Ansprüche 4-6, wobei
die Befestigungsfläche (49) des Kontaktelements (40) senkrecht zur optischen Achse (20) des Linsenelements (10) verläuft, wenn das Linsenelement (10) in dem Kontaktelement (40) befestigt ist.

8. System nach einem der Ansprüche 1-3 oder Kontaktvorrichtung (5) nach einem der Ansprüche 4-7, wobei
das mindestens eine Vorsprungselement (25) des Linsenelements (10) eine im Wesentlichen vollständig um die optische Achse (20) des Linsenelements (10) umlaufende Wulst umfasst oder ist oder das mindestens eine Vorsprungselement (25) mehrere um die optische Achse (20) der Linse herum zueinander beabstandet angeordnete Vorsprungselemente (25) aufweist.

9. System nach einem der Ansprüche 1-3 oder Kontaktvorrichtung (5) nach einem der Ansprüche 4-8, wobei
das Ausgleichselement (50) in einem Querschnitt senkrecht zur optischen Achse (20) des Linsenelements (10) im Wesentlichen kreisringförmig ausgebildet ist, wenn das Linsenelement (10) in dem Kontaktelement (40) befestigt ist.

10. System nach einem der Ansprüche 1-3 oder Kontaktvorrichtung (5) nach einem der Ansprüche 4-9, wobei
das Kontaktelement (40) mehrere Halteelemente (42-47) umfasst, die, insbesondere äquidistant zueinander, um die optische Achse (20) der Linse herum beabstandet zueinander angeordnet sind, wenn das Linsenelement (10) in dem Kontaktelement (40) befestigt ist.

11. System nach einem der Ansprüche 1-3 oder Kontaktvorrichtung (5) nach einem der Ansprüche 4-10, wobei
das eine oder die mehreren Halteelemente (42-47) des Kontaktelements (40) derart flexibel ausgebildet sind, dass das eine oder die mehreren Halteelemente (42-47) mechanisch beweglich zum Einrasten des mindestens einen Vorsprungs des Linsenelements (10) unter dem Halteelement (42-47) oder den Halteelementen (42-47) zum Bilden der Schnappverbindung ausgebildet ist/sind.

12. System nach einem der Ansprüche 1-3 oder Kontaktvorrichtung (5) nach einem der Ansprüche 4-11, wobei
das Ausgleichselement (50) eine der jeweiligen Bauart der Kontaktvorrichtung (5) zugeordnete Markierung und/oder Farbe aufweist.

13. Verfahren zum Herstellen einer Kontaktvorrichtung (5) zur Fixierung der relativen geometrischen Lage eines Patientenauges zu einem Laser-Applikator eines ophthalmologischen Lasertherapiesystems, wobei die Kontaktvorrichtung (5) ein Kontaktelement (40) und ein Linsenelement (10) aufweist, wobei das Verfahren folgende Schritte umfasst:
Bereitstellen eines Kontaktelements (40) mit einem oder mehreren Halteelementen (42-47);
Bereitstellen eines Linsenelements (10),
wobei das Linsenelement (10) eine Augenfläche (30) zum Berühren des Patientenauges oder zum luftdichten Begrenzen eines Hohlraums zwischen dem Linsenelement (10) und dem Patientenauge aufweist,
wobei das Linsenelement (10) mindestens ein Vorsprungselement (25) zum Untergreifen des einen oder der mehreren Halteelemente (42-47) des Kontaktelements (40) zum klebstofflosen Befestigen des Linsenelements (10) in dem Kontaktelement (40) mittels einer Schnappverbindung aufweist,
wobei das Linsenelement (10) eine auf einer der Augenfläche (30) zugewandten Unterseite des mindestens einen Vorsprungselements (25) ausgebildete Linsenkontaktfläche (15) aufweist;
und
klebstoffloses Befestigen des Linsenelements (10) in dem Kontaktelement (40) mittels einer Schnappverbindung, indem das mindestens eine Vorsprungselement (25) unter dem oder den Halteelementen (42-47) derart eingeschnappt wird, dass die Linsenkontaktfläche (15) mittels des mindestens einen Vorsprungselements (25) und des einen Halteelements (42-47) oder der mehreren Halteelementen (42-47) gegen ein auf einer Befestigungsfläche (49) des Kontaktelements (40) angeordnetes elastisches Ausgleichselement (50) zum Verringern der durch das eine Halteelement (42-47) oder die mehreren Halteelemente (42-47) auf das Linsenelement (10) wirkenden Kräfte gedrückt wird.

14. Verfahren nach Anspruch 13, wobei
das Kontaktelement (40) umfassend das flexible Ausgleichselement (50) mittels Mehrkomponenten-Spritzgussverfahren, insbesondere Zweikomponenten-Spritzgussverfahren, hergestellt wird.

15. Verfahren nach Anspruch 13 oder 14, wobei
das eine oder die mehreren Halteelemente (42-47) des Kontaktelements (40) mechanisch, insbesondere von der optischen Achse (20) des Linsenelements (10) weg, reversibel bewegt werden zum Einführen eines Teils des mindestens einen Vorsprungs des Linsenelements (10) zwischen das Halteelement (42-47) oder die Halteelemente (42-47) des Kontaktelements (40) und die Befestigungsfläche (49) des Kontaktelements (40) zur Bildung der Schnappverbindung.

## Claims

1. System comprising
a contact element (40) for fixing the relative geometric position of a patient's eye to a laser applicator of an ophthalmic laser therapy system,
and
a lens element (10) for insertion into the contact element (40),
wherein the contact element (40) is configured for receiving the lens element (10) at a predetermined position,
wherein the lens element (10) has an eye surface (30) for contacting the patient's eye or for airtightly delimiting a cavity between the lens element (10) and the patient's eye,
wherein the lens element (10) has at least one projection element (25) for engaging under one or more retaining elements (42-47) of the contact element (40) for adhesive-free fastening of the lens element (10) in the contact element (40) by means of a snap connection,
wherein the lens element (10) has a lens contact surface (15) formed on an underside of the projection element (25) facing the eye surface (30),
wherein the lens element (10) and the contact element (40) are configured in such a way that the lens element (10) can be fastened in the contact element (40) by means of the snap connection such that the lens contact surface (15) is pressed by means of the at least one projection element (25) and the one retaining element (42-47) or the plurality of retaining elements (42-47) against an elastic compensating element (50) arranged on an attachment surface (49) of the contact element (40) for reducing the forces acting on the lens element (10) by the one retaining element (42-47) or the plurality of retaining elements (42-47).

2. System according to claim 1, wherein
the lens element (10) and the contact element (40) are configured such that the lens element (10) can be fastened in the contact element (40) such that the compensating element (50) is arranged completely circumferentially around the optical axis (20) of the lens element (10).

3. System according to claim 1 or 2, wherein
the compensating element (50) is configured for airtight sealing of the area between the lens contact surface (15) and the attachment surface (49).

4. Contact device (5),
wherein the contact device (5) comprises a system according to any one of claims 1-3,
wherein the contact device (5) is configured for fixing the relative geometric position of the patient's eye to the laser applicator of the ophthalmic laser therapy system,
wherein the lens element (10) is firmly connected to the contact element (40) without adhesive by means of the snap connection,
wherein the lens element (10) is fastened in the contact element (40) by means of the snap connection in such a way that the lens contact surface (15) is pressed by means of the at least one projection element (25) and the one retaining element (42-47) or the plurality of retaining elements (42-47) against the elastic compensating element (50) arranged on the attachment surface (49) of the contact element (40) for reducing the forces acting on the lens element (10) by the one retaining element (42-47) or the plurality of retaining elements (42-47).

5. Contact device (5) according to claim 4, wherein
the lens element (10) is fastened in the contact element (40) in such a way that the compensating element (50) is arranged completely circumferentially around the optical axis (20) of the lens element (10).

6. System according to any one of claims 1-3 or contact device (5) according to claim 4 or claim 5, wherein
the lens contact surface (15) runs perpendicularly to the optical axis (20) of the lens element (10).

7. System according to any one of claims 1-3 or contact device (5) according to any one of claims 4-6, wherein
the attachment surface (49) of the contact element (40) runs perpendicularly to the optical axis (20) of the lens element (10), when the lens element (10) is fastened in the contact element (40).

8. System according to any one of claims 1-3 or contact device (5) according to any one of claims 4-7, wherein
the at least one projection element (25) of the lens element (10) comprises or is a bulge extending substantially completely around the optical axis (20) of the lens element (10) or the at least one projection element (25) comprises a plurality of projection elements (25) arranged at a distance from one another around the optical axis (20) of the lens.

9. System according to any one of claims 1-3 or contact device (5) according to any one of claims 4-8, wherein
the compensating element (50) is substantially circular in a cross-section perpendicular to the optical axis (20) of the lens element (10), when the lens element (10) is fastened in the contact element (40).

10. System according to any one of claims 1-3 or contact device (5) according to any one of claims 4-9, wherein
the contact element (40) comprises a plurality of retaining elements (42-47) which are arranged at a distance from one another, in particular equidistant from one another, around the optical axis (20) of the lens, when the lens element (10) is fastened in the contact element (40).

11. System according to any one of claims 1-3 or contact device (5) according to any one of claims 4-10, wherein
the one or more retaining elements (42-47) of the contact element (40) is/are flexible such that the one or more retaining elements (42-47) is/are mechanically movable to engage the at least one projection of the lens element (10) under the retaining element (42-47) or the retaining elements (42-47) to form the snap connection.

12. System according to any one of claims 1-3 or contact device (5) according to any one of claims 4-11, wherein
the compensating element (50) has a marking and/or color assigned to the respective layout of the contact device (5).

13. A method of manufacturing a contact device (5) for fixing the relative geometric position of a patient's eye to a laser applicator of an ophthalmic laser therapy system, wherein the contact device (5) comprises a contact element (40) and a lens element (10), wherein the method comprises the following steps:
providing a contact element (40) with one or more retaining elements (42-47);
providing a lens element (10),
wherein the lens element (10) has an eye surface (30) for contacting the patient's eye or for airtightly delimiting a cavity between the lens element (10) and the patient's eye,
wherein the lens element (10) has at least one projection element (25) for engaging under the one or more retaining elements (42-47) of the contact element (40) for adhesive-free fastening of the lens element (10) in the contact element (40) by means of a snap connection,
wherein the lens element (10) has a lens contact surface (15) formed on an underside of the at least one projection element (25) facing the eye surface (30);
and
adhesive-free fastening of the lens element (10) in the contact element (40) by means of a snap connection by snapping in the at least one projection element (25) under the retaining element or elements (42-47) in such a way that the lens contact surface (15) is pressed by means of the at least one projection element (25) and the one retaining element (42-47) or
the plurality of retaining elements (42-47) against an elastic compensating element (50) arranged on an attachment surface (49) of the contact element (40) for reducing the forces acting on the lens element (10) by the one retaining element (42-47) or the plurality of retaining elements (42-47).

14. Method according to claim 13, wherein
the contact element (40) comprising the flexible compensating element (50) is manufactured by means of a multi-component injection molding process, in particular a two-component injection molding process.

15. Method according to claim 13 or 14, wherein
the one or more retaining elements (42-47) of the contact element (40) are reversibly moved mechanically, in particular away from the optical axis (20) of the lens element (10), for inserting a part of the at least one projection of the lens element (10) between the retaining element (42-47) or the retaining elements (42-47) of the contact element (40) and the attachment surface (49) of the contact element (40) for forming the snap connection.

## Revendications

1. Système comprenant
un élément de contact (40) pour fixer la position géométrique relative de l'œil d'un patient par rapport à un applicateur laser d'un système de thérapie laser ophtalmologique,
et
un élément de lentille (10) destiné à être inséré dans l'élément de contact (40),
dans lequel l'élément de contact (40) étant conçu pour recevoir l'élément de lentille (10) dans une position prédéterminée,
dans lequel l'élément de lentille (10) a une surface d'oeil (30) pour toucher l'oeil du patient ou pour délimiter de manière étanche à l'air une cavité entre l'élément de lentille (10) et l'oeil du patient,
dans lequel l'élément de lentille (10) a au moins un élément en saillie (25) destiné à s'engager sous un ou plusieurs éléments de retenue (42-47) de l'élément de contact (40) pour fixer sans colle l'élément de lentille (10) dans l'élément de contact (40) au moyen d'une liaison par encliquetage,
dans lequel l'élément de lentille (10) a une surface de contact de lentille (15) formée sur une face inférieure de l'élément saillant (25) tournée vers la surface de l'oeil (30),
dans lequel l'élément de lentille (10) et l'élément de contact (40) étant formés de telle sorte que l'élément de lentille (10) peut être fixé dans l'élément de contact (40) au moyen de la connexion par encliquetage, en ce que la surface de contact de lentille (15) est pressée, au moyen du au moins un élément en saillie (25) et du un élément de retenue (42-47) ou des plusieurs éléments de retenue (42-47), contre un élément de compensation élastique (50) disposé sur une surface de fixation (49) de l'élément de contact (40) pour réduire les forces agissant sur l'élément de lentille (10) par le un élément de retenue (42-47) ou les plusieurs éléments de retenue (42-47).

2. Système selon la revendication 1, dans lequel
l'élément de lentille (10) et l'élément de contact (40) sont conçus de telle sorte que l'élément de lentille (10) peut être fixé dans l'élément de contact (40) de telle sorte que l'élément de compensation (50) est disposé de manière à tourner complètement autour de l'axe optique (20) de l'élément de lentille (10).

3. Système selon la revendication 1 ou 2, dans lequel
l'élément de compensation (50) est conçu pour rendre étanche à l'air la zone située entre la surface de contact de la lentille (15) et la surface de fixation (49).

4. Dispositif de contact (5),
dans lequel le dispositif de contact (5) comprend un système selon l'une des revendications 1-3,
dans lequel le dispositif de contact (5) est conçu pour fixer la position géométrique relative de l'œil du patient par rapport à l'applicateur laser du système de thérapie laser ophtalmologique,
dans lequel l'élément de lentille (10) étant relié solidement sans colle à l'élément de contact (40) au moyen de la liaison par encliquetage,
dans lequel l'élément de lentille (10) étant fixé dans l'élément de contact (40) au moyen de la liaison par encliquetage, en ce que la surface de contact de lentille (15) est pressée, au moyen du au moins un élément en saillie (25) et du un élément de retenue (42-47) ou des plusieurs éléments de retenue (42-47), contre l'élément de compensation élastique (50) disposé sur la surface de fixation (49) de l'élément de contact (40) pour réduire les forces agissant sur l'élément de lentille (10) par le un élément de retenue (42-47) ou les plusieurs éléments de retenue (42-47).

5. Dispositif de contact (5) selon la revendication 4, dans lequel
l'élément de lentille (10) est fixé dans l'élément de contact (40) de telle sorte que l'élément de compensation (50) est disposé de manière à tourner complètement autour de l'axe optique (20) de l'élément de lentille (10).

6. Système selon l'une des revendications 1-3 ou dispositif de contact (5) selon la revendication 4 ou la revendication 5, dans lequel
la surface de contact de lentille (15) est perpendiculaire à l'axe optique (20) de l'élément de lentille (10).

7. Système selon l'une quelconque des revendications 1-3 ou dispositif de contact (5) selon l'une des revendications 4-6, dans lequel
la surface de fixation (49) de l'élément de contact (40) est perpendiculaire à l'axe optique (20) de l'élément de lentille (10) lorsque l'élément de lentille (10) est fixé dans l'élément de contact (40).

8. Système selon l'une quelconque des revendications 1-3 ou dispositif de contact (5) selon l'une des revendications 4-7, dans lequel
le au moins un élément saillant (25) de l'élément de lentille (10) comprend ou est un bourrelet faisant sensiblement tout le tour de l'axe optique (20) de l'élément de lentille (10) ou le au moins un élément saillant (25) comprend plusieurs éléments saillants (25) espacés les uns des autres autour de l'axe optique (20) de la lentille.

9. Système selon l'une des revendications 1-3 ou dispositif de contact (5) selon l'une des revendications 4-8, dans lequel
l'élément de compensation (50) a une forme sensiblement annulaire dans une section transversale perpendiculaire à l'axe optique (20) de l'élément de lentille (10) lorsque l'élément de lentille (10) est fixé dans l'élément de contact (40).

10. Système selon l'une des revendications 1-3 ou dispositif de contact (5) selon l'une des revendications 4-9, dans lequel
l'élément de contact (40) comprend une pluralité d'éléments de maintien (42-47) espacés les uns des autres, en particulier à équidistance les uns des autres, autour de l'axe optique (20) de la lentille lorsque l'élément de lentille (10) est fixé dans l'élément de contact (40).

11. Système selon l'une quelconque des revendications 1-3 ou dispositif de contact (5) selon l'une des revendications 4-10, dans lequel
l'un élément de retenue ou les éléments de retenue (42-47) de l'élément de contact (40) sont flexibles de telle sorte que l'un élément de retenue ou les éléments de retenue (42-47) sont mécaniquement mobiles pour encliqueter la au moins une saillie de l'élément de lentille (10) sous l'élément de retenue (42-47) ou les éléments de retenue (42-47) pour former la connexion encliquetée.

12. Système selon l'une des revendications 1-3 ou dispositif de contact (5) selon l'une des revendications 4-11, dans lequel
l'élément de compensation (50) a un marquage et/ou une couleur associés au type de construction respectif du dispositif de contact (5).

13. Procédé de fabrication d'un dispositif de contact (5) pour fixer la position géométrique relative de l'oeil d'un patient par rapport à un applicateur laser d'un système de thérapie laser ophtalmologique, dans lequel le dispositif de contact (5) comprend un élément de contact (40) et un élément de lentille (10), dans lequel le procédé comprend les étapes suivantes:
fournir un élément de contact (40) avec un ou plusieurs éléments de maintien (42-47);
fournir un élément de lentille (10),
dans lequel l'élément de lentille (10) a une surface d'oeil (30) pour entrer en contact avec l'oeil du patient ou pour délimiter de manière étanche à l'air une cavité entre l'élément de lentille (10) et l'oeil du patient,
dans lequel l'élément de lentille (10) a au moins un élément en saillie (25) destiné à s'engager sous l'un élément de retenue ou les plusieurs éléments de retenue (42-47) de l'élément de contact (40) pour fixer sans colle l'élément de lentille (10) dans l'élément de contact (40) au moyen d'une liaison par encliquetage,
dans lequel l'élément de lentille (10) a une surface de contact de lentille (15) formée sur une face inférieure de l'au moins un élément en saillie (25) tournée vers la surface de l'oeil (30); et
fixer sans colle de l'élément de lentille (10) dans l'élément de contact (40) au moyen d'une connexion par encliquetage, en encliquetant le au moins un élément saillant (25) sous le ou les éléments de retenue (42-47) de telle sorte, que la surface de contact de la lentille (15) est pressée au moyen du au moins un élément en saillie (25) et du un élément de retenue (42-47) ou des plusieurs éléments de retenue (42-47) contre un élément de compensation élastique (50) disposé sur une surface de fixation (49) de l'élément de contact (40) pour réduire les forces agissant sur l'élément de lentille (10) par le un élément de retenue (42-47) ou les plusieurs éléments de retenue (42-47).

14. Procédé selon la revendication 13, dans lequel
l'élément de contact (40) comprenant l'élément de compensation flexible (50) est fabriqué au moyen d'un procédé de moulage par injection à plusieurs composants, en particulier d'un procédé de moulage par injection à deux composants.

15. Procédé selon la revendication 13 ou 14, dans lequel
le un élément de retenue ou les plusieurs éléments de retenue (42-47) de l'élément de contact (40) sont déplacés mécaniquement de manière réversible, en particulier en s'éloignant de l'axe optique (20) de l'élément de lentille (10), pour introduire une partie de la au moins une saillie de l'élément de lentille (10) entre l'élément de retenue (42-47) ou les éléments de retenue (42-47) de l'élément de contact (40) et la surface de fixation (49) de l'élément de contact (40) pour former la liaison par encliquetage.
